# FASCICULE DE BREVET EUROPEEN

(11) **EP 0 583 998 B1**
(45) Date de publication et mention de la délivrance du brevet: **20.01.1999**
(21) Numéro de dépôt: 93401843.3
(22) Date de dépôt: 16.07.1993
(51) Int. Cl.: A61K 39/205

(54) **Vaccin antirabique avirulent**
Avirulenter Tollwut-Impfstoff
Avirulent antirabies vaccine

(30) Priorité: 20.07.1992 FR 9208947
(43) Date de publication de la demande: 23.02.1994
(73) Titulaire: VIRBAC S.A., F-06516 Carros Cedex (FR)
(72) Inventeur: Benejean, Jacqueline, F-91380 Chilly Mazarin (FR); Flamand, Anne, F-91190 Gif Sur Yvette (FR); Tuffereau, Marie-Christine, F-75014 Paris (FR); Coulon, Patrice, F-91120 Palaiseau (FR); Lafay, Florence, F-78000 Versailles (FR)
(74) Mandataire: Gillard, Marie-Louise

(56) Documents cités:
- EP-A- 0 202 142
- EP-A- 0 350 398
- VIROLOGY vol. 172, 1989, NEW YORK,USA pages 206 - 212 TUFFEREAU ET AL 'ARGININE OR LYSINE IN POSITION 333 OF ERA AND CVS GLYCOPROTEIN IS NECESSARY FOR RABIES VIRULENCE IN ADULT MICE'
- ANNALES DE L'INSTITUT PASTEUR / VIROLOGIE vol. 136E, 1985, AMSTERDAM,NL pages 363 - 372 FLAMAND ET AL 'LA RAGE:EFFET,SUR LA VIRULENCE,DE MUTATIONS LOCALISEES DANS LE SITE III DE LA GLYCOPROTEINE'
- PROCEEDINGS OF THE NATIONAL ACADEMY OF SCIENCES,USA vol. 80, 1983, WASHINGTON D.C.,USA pages 70 - 74 DIETZSCHOLD ET AL 'CHARACTERIZATION OF AN ANTIGENIC DETERMINANT OF THE GLYCOPROTEIN THAT CORRELATES WITH PATHOGENICITY OF RABIES VIRUS'

## Description

La présente invention concerne un nouveau vaccin antirabique.

Le virus rabique est un rhabdovirus constitué de cinq protéines, dont une protéine externe, la glycoprotéine, qui déclenche la synthèse d'anticorps neutralisants chez les animaux inoculés. L'injection de la glycoprotéine purifiée protège l'animal contre une surinfection. Les souches du virus rabique les plus utilisées, notamment la souche CVS et la souche ERA, dont dérivent les souches SAD, telles que les souches SAD Berne et SAD B19 sont décrites dans "Rabies Viruses" par H.F. Clark et T.J. Wiktor - Strains of Human Viruses édité par Majer et Plotkin Karger, Bâle, 1972, p. 177-182. La séquence en acides aminés de la glycoprotéine de la souche CVS a été décrite par Yelverton et al. "Rabies virus glycoprotein analogs : biosynthesis in Escherichia coli" Science 219, 614-620.

Cette glycoprotéine comporte deux sites antigéniques majeurs distincts liés à la neutralisation du virus (sites II et III). Le site III en position 330-340 contient l'arginine 333 qui détermine la virulence de cette souche. Il a été trouvé, pour qu'il y ait virulence virale, qu'un acide aminé à charge positive (arginine ou lysine) doit être présent en position 333 de la glycoprotéine (Tuffereau et al., Virology 172, 206-212 (1989). Ce document décrit également qu'il existe des mutants avirulents possédant en position 333 une glycine, une glutamine, une méthionine ou une sérine.

La séquence en acides aminés de la glycoprotéine de la souche SAD Berne n'a pas été entièrement établie. Toutefois, on a pu déterminer que le site antigénique III de la glycoprotéine de la souche SAD Berne est identique à celui de la glycoprotéine de la souche CVS.

Les vaccins antirabiques actuellement utilisés sont soit des vaccins fabriqués à partir de virus inactivés, soit des vaccins constitués de souches virales dont la virulence a été atténuée, soit des virus recombinants (par exemple la vaccine).

L'inactivation des virus peut être réalisée par différents procédés, notamment par des procédés chimiques, tels que le traitement au formol ou à la β-propiolactone.

L'inconvénient majeur de ce procédé de fabrication de vaccins est la manipulation de souches virulentes qui nécessite des conditions de mise en oeuvre très strictes et présente des risques de contamination du personnel de fabrication.

D'autre part, les vaccins inactivés administrés par voie orale n'ont pas de pouvoir protecteur.

L'atténuation de la virulence des souches virales est une technique bien connue; elle peut être réalisée par exemple par passages successifs des souches virales sur un hôte différent de l'espèce vecteur (lapin ou souris par exemple) ou en culture de cellules. On obtient ainsi des souches désadaptées à l'hôte d'origine et donc moins pathogènes pour celui-ci tout en gardant leur capacité vaccinante.

Les souches SAD, telles que les souches SAD B19 et SAD Berne accessibles au public, sont des souches atténuées qui ont déjà été testées en Europe pour la vaccination des renards. Elles peuvent être mélangées aux appâts pour une administration par voie orale. Toutefois, ces souches se sont montrées pathogènes pour d'autres espèces animales et l'homme. Il existe donc un risque potentiel de contamination qui diminue considérablement l'intérêt de ces souches pour la vaccination orale.

En effet, par exemple, l'administration par voie orale de la souche SAD Berne à un lot de 23 rongeurs sauvages, choisis parmi Apodemus flavicolus et sylvaticus, Arvicola terrestris, Clethrionomys clareolus, Minotus agresti a provoqué la mort par la rage de deux animaux.

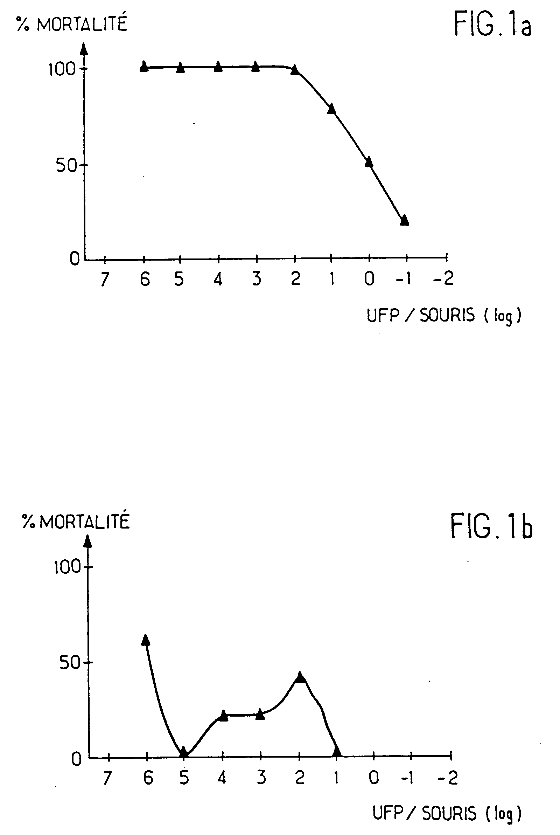

Des essais sur la souris ont également montré que la souche SAD Berne est pathogène pour cette espèce aussi bien par voie intra-cérébrale que par voie intra-musculaire, comme le montrent les courbes des figures 1a et 1b ci-jointes qui sont respectivement:
- Figure 1a: Courbe de mortalité chez les souris adultes en fonction des doses administrées (en unités formant plage) par voie intra-cérébrale.
- Figure 1b : Courbe de mortalité chez les souris adultes en fonction des doses administrées (en unités formant plage) par voie intra-musculaire.

Par voie orale, on a noté chez la souris une mortalité de 10 % à la dose de 10^{5,5} UFP.

La souche SAD Berne telle quelle, utilisée pour des campagnes de vaccination des renards, présente donc un risque pour la faune sauvage et l'homme. Il en est de même de la souche SAD B19.

Pour pallier cet inconvénient, on a déjà proposé un vaccin antirabique avirulent. Ce vaccin, décrit dans la demande de Brevet EP 350 398, est constitué d'un mutant avirulent d'une souche SAD du virus de la rage qui est caractérisé par le fait qu'il possède à la place de l'arginine 333 de la glycoprotéine un acide aminé autre que la lysine, par exemple la glycine, l'isoleucine ou la sérine.

Ce mutant dérive par le changement d'un seul nucléotide dans le codon de l'arginine 333.

Malheureusement, ce mutant peut, par une simple mutation réverse, redonner la souche parentale.

Ce vaccin qui est utilisé pour l'administration par voie orale n'est donc pas totalement sans danger pour les autres espèces animales.

La présente invention concerne un vaccin efficace qui permet de pallier les inconvénients ci-dessus.

Le vaccin selon l'invention est constitué d'un mutant avirvlent d'une souche SAD du virus rabique qui est caractérisé par le fait qu'il possède à la place de l'arginine 333 de la glycoprotéine un acide aminé naturel dont le codon diffère de ceux codant pour l'arginine par deux nucléotides.

L'invention a également pour objet les mutants avirulents d'une souche SAD du virus rabique, qui possèdent, à la place de l'arginine en 333 de la glycoprotéine, un acide aminé dont le codon diffère de ceux de l'arginine par deux nucléotides.

L'invention concerne aussi un procédé pour l'obtention des mutants avirvlents définis ci-dessus. Ce procédé consiste :
1/ à sélectionner à partir d'une souche SAD du virus rabique, les mutants qui ne sont pas neutralisés par un anticorps monoclonal neutralisant ladite souche SAD, mais ne neutralisant pas la souche TAG1 définie ci-après ;
2/ à isoler, par séquençage de la région 333 de la glycoprotéine des mutants sélectionnés à l'étape 1/, un mutant qui possède en 333 une lysine ;
3/ à préparer un anticorps monoclonal qui neutralise à la fois ladite souche SAD et le mutant obtenu à l'étape 2/, mais ne neutralise pas la souche TAG1 ;
4/ à procéder à une seconde sélection parmi les mutants obtenus à l'étape 1/ à l'aide de l'anticorps monoclonal préparé à l'étape 3/.

Les anticorps monoclonaux mis en oeuvre pour sélectionner les mutants selon l'invention sont obtenus par fusion de cellules de myélome et de cellules produisant des anticorps antiviraux selon la technique de l'hybridation décrite par KOHLER et MILSTEIN dans NATURE, Vol. 256, 495-497 (1975), technique maintenant bien connue de l'homme de l'art.

Selon cette technique on peut fusionner des cellules provenant d'espèces différentes, toutefois, il est avantageux d'utiliser des cellules provenant de la même espèce animale. Par exemple on utilise de préférence d'une part des cellules de myélome de souris et d'autre part des cellules de rate de souris préalablement immunisées avec une souche du virus de la rage selon le protocole défini ci-après.

Sous son aspect général, ce procédé d'hybridation décrit en référence aux cellules de souris, comprend les étapes suivantes:
1) d'immunisation de souris avec une quantité donnée de virus inactivé à la β-propriolactone ,
2) de prélèvement de la rate des souris immunisées et séparation des splénocytes;
3) de fusion des splénocytes ainsi obtenus avec des cellules de myélome de souris en présence d'un promoteur de fusion;
4) de culture des cellules hybrides obtenues dans un milieu sélectif sur lequel ne se développent pas les cellules de myélomes non fusionnées, et en présence d'éléments appropriés;
5) de sélection des cellules produisant l'anticorps désiré et clonage de ces cellules.

Le protocole d'immunisation comprend l'injection à des souris Balb-C de 100 µg de virus CV5 inactivé à la β-propiolactone par voie intrapéritonéale avec adjuvant complet de FREUND et un rappel par voie intraveineuse 4 jours avant la fusion après une période de repos de 1 mois.

Les splénocytes des souris immunisées sont récupérés après prélèvement de la rate selon le mode opératoire classique.

Les cellules de myélome de souris utilisées pour l'obtention des anticorps monoclonaux neutralisants sont les cellules de myélome de souris Balb-C provenant de la lignée SP₂0_{.} Ces cellules de myélome ont été sélectionnées pour leur sensibilité à l'aminoptérine et cultivées sur un milieu approprié, tel que le milieu essentiel d'Eagle modifié par DULBECCO (Dulbecco Modified Eagle medium) dénommé ci-après milieu DMEM, auquel on a ajouté 15 % de sérum du poulain.

La fusion des cellules de myélome avec les splénocytes a été réalisée par mélange de 5.10⁷ cellules de myélome avec 5.10⁷ cellules de rate de souris immunisées, en présence d'un promoteur de fusion, tel que par exemple un polyéthylène glycol.

Après incubation à 37° C, les cellules sont lavées dans le milieu DMEM et remises en suspension, puis cultivées sur un milieu sélectif approprié uniquement pour la croissance des cellules hybrides. Un tel milieu contient de l'hypoxanthine, de l'aminoptérine et de la thymidine.

On sélectionne ensuite les surnageants des cultures, 7 à 20 jours après la fusion, en mettant en contact les surnageants avec une suspension de virus CVS et en retenant les anticorps qui neutralisent ladite suspension.

Par "anticorps neutralisant un virus", on désigne les anticorps qui, mis en présence d'une suspension dudit virus, inhibent la virulence de celui-ci.

Le pouvoir neutralisant des anticorps monoclonaux obtenus ci-dessus est déterminé selon un procédé classique bien connu de l'homme de métier. Ce procédé consiste à mettre en présence 100 µl de suspension de virus contenant 1000 UFP de virus et 100 µl de surnageant de culture d'hybridome, à infecter une culture de cellules avec ce mélange et après 4 jours d'incubation à dénombrer les plages de lyse sous agar selon la méthode décrite par BUSSEREAU et al., 1982, J. Virol. Méth. Vol. 4 p 277-282. L'anticorps est neutralisant lorsqu'il inhibe toute formation de plage sous agar dans les conditions décrites ci-dessus.

Parmi ces anticorps on retient ensuite les anticorps qui ne neutralisent pas le mutant avirulent TAG1 dérivant de la souche CVS, déposé à la Collection Nationale de Cultures de Micro-organismes (C.N.C.M) INSTITUT PASTEUR - FRANCE le 12 avril 1985 sous le N° I-433.

Les anticorps monoclonaux résultants, qui neutralisent donc la souche CVS mais ne neutralisent pas le mutant avirulent TAG1 permettent de sélectionner des mutants avirulents à partir de toute souche de virus de la rage, qui est neutralisée par ces anticorps monoclonaux.

Les anticorps monoclonaux ainsi obtenus sont des anticorps neutralisant également les souches SAD du virus rabique. Ils conviennent donc pour effectuer la première sélection du procédé de l'invention.

Le séquençage de la région 333 de la glycoprotéine des mutants sélectionnés à l'étape 1/ est réalisé selon la méthode classique bien connue de l'homme de métier [SANGER et al., 1977, Proc. Nat., Acad. Sci. USA, vol. 74 p 5463-5467].

Ce séquençage permet d'isoler un mutant qui possède, en position 333 de la glycoprotéine une lysine ; ce mutant est dénommé ci-après "mutant SK".

Le codon (AAA) de cet acide aminé (lysine) diffère de celui de l'arginine en position 333 de la souche SAD par un seul nucléotide, le codon de l'arginine en position 333 de la souche SAD étant AGA.

On procède ensuite à la préparation de l'anticorps monoclonal neutralisant à la fois la souche SAD et le mutant obtenu ci-dessus.

Cet anticorps monoclonal est obtenu en retenant, parmi les anticorps monoclonaux neutralisant la souche SAD et ne neutralisant pas TAG1, celui qui neutralise également le mutant lysine ou mutant SK obtenu ci-dessus.

Cet anticorps monoclonal permet ensuite de procéder à la seconde sélection (étape 4) du procédé de l'invention.

Le pouvoir pathogène des mutants résultant de cette seconde sélection est ensuite testé par injection intra-cérébrale de 10⁵ UFP à des souris adultes. Les mutants qui ne tuent pas à cette dose et par cette voie d'injection sont considérés comme avirulents.

Les mutants selon l'invention sont des doubles mutants avirulents d'une souche SAD, telle que notamment la souche SAD Berne, résultant de deux sélections successives à l'aide des anticorps monoclonaux définis ci-dessus.

Les mutants selon l'invention peuvent en outre contenir d'autres mutations, comme par exemple, une mutation conférant la résistance à un anticorps monoclonal spécifique du site antigénique II, ce qui permet de reconnaître un éventuel revertant de virulence des souches SAD utilisées pour la vaccination orale des renards.

Les mutants selon l'invention peuvent être multipliés sur cellules de rein de hamster nouveau-nés BHK 21, en présence de milieu GEM (milieu essentiel minimum-modification Glasgow commercialisé par FLOW) et de 2 % de sérum de veau à 33° C et en atmosphère humide avec 5% de CO₂.

On les titre selon les méthodes habituelles par exemple par détermination de la dose létale 50 (DL₅₀) chez les souriceaux, par immunofluorescence ou dénombrement des plages de lyse sous agar. Ils peuvent être conservés à -70°C.

L'analyse de la séquence en nucléotides de la glycoprotéine de ces mutants a révélé que le codon de l'acide aminé en 333 diffère par au moins deux nucléotides de tous les codons possibles de l'arginine. Parmi les mutants qui remplissent cette condition, on préfère tout particulièrement le mutant possédant en 333 un acide glutamique, car il se multiplie bien en culture de cellules, il est moins pathogène pour les souriceaux nouveau-nés et il a un bon pouvoir protecteur.

Le double mutant portant un acide glutamique dont le codon est: GAA à la place de l'arginine en 333, obtenu selon le procédé ci-dessus à partir de la souche SAD Berne et dénommé ci-après SAG2, a été déposé à la Collection Nationale de Cultures de Micro-organismes (C.N.C.M) INSTITUT PASTEUR - FRANCE le 9 juillet 1992 sous le N°I-1238. Ce mutant contient une autre mutation, à savoir la résistance à un anticorps monoclonal spécifique du site antigénique II, ce qui sert de marqueur additionnel de la souche.

L'invention va être maintenant décrite plus en détail en référence, au mutant SAG2 sans pour autant limiter la portée de celle-ci à ce seul mutant.

### A - Tests de stabilité génétique de la souche au cours de passages en cerveaux de souriceaux.

10³ UFP de SAG2 ont été injectés à 6 souriceaux de 4 jours. Quand les animaux ont été malades (J6) ils ont été sacrifiés. Six broyats individuels ont été effectués ; chaque broyat a été titré et 30 µl d'une dilution au 1/10 ont été injectés à 3 souris adultes (contrôle de pathogénie) et à un souriceau (passage suivant). Les 6 souriceaux ont ainsi permis de faire 6 séries indépendantes de 3 passages.

Les titres des cerveaux en UFP/ml sont donnés dans le tableau suivant :

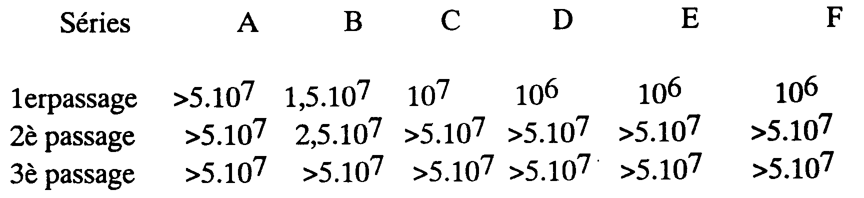

Tous les adultes injectés (soit 54 souris) après les 1er, 2è ou 3è passages ont survécu montrant l'absence de réversion.

### B- Pouvoir protecteur de SAG2

Le mutant SAG2 a été injecté par voie intra-cérébrale à des souris et on a déterminé le pouvoir protecteur de ce mutant par épreuve intra-musculaire de 100 DL 50 de la souche CVS.

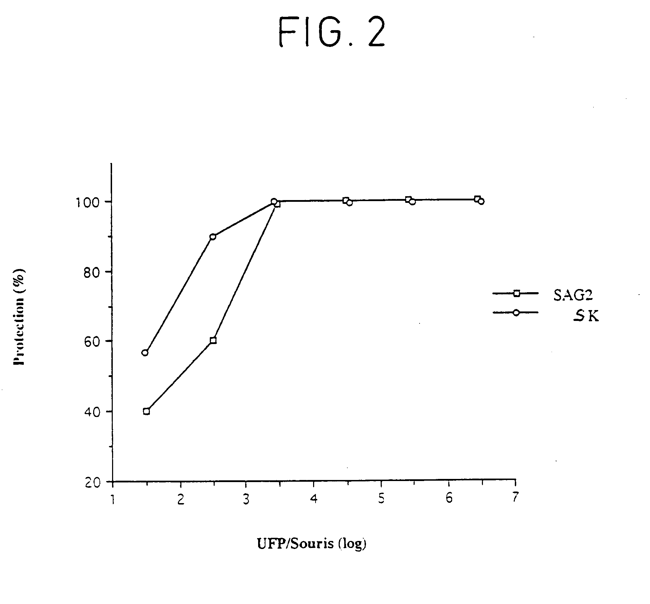

Les résultats obtenus sont reportés sur la figure 2 qui est un graphe donnant le pouvoir protecteur (%) en ordonnées en fonction de la quantité du mutant injecté exprimé en UFP/souris (log). On a répété le même test avec le mutant SK possédant en 333 une lysine.

On a constaté que le pouvoir protecteur de SK et de SAG2 était de 100 % à partir de 10⁴ UFP/souris.

### C- Pathogénie de SAG2 par voie intra-cérébrale

On a injecté le mutant SAG2 à des souris à des doses allant de 10^{- 0,5} à 10⁶ UFP/souris et on n'a pas constaté de mortalité sur une période de 28 jours.

Parallèlement, on a effectué la même expérience avec le virus SAD BERNE ou le mutant SK.

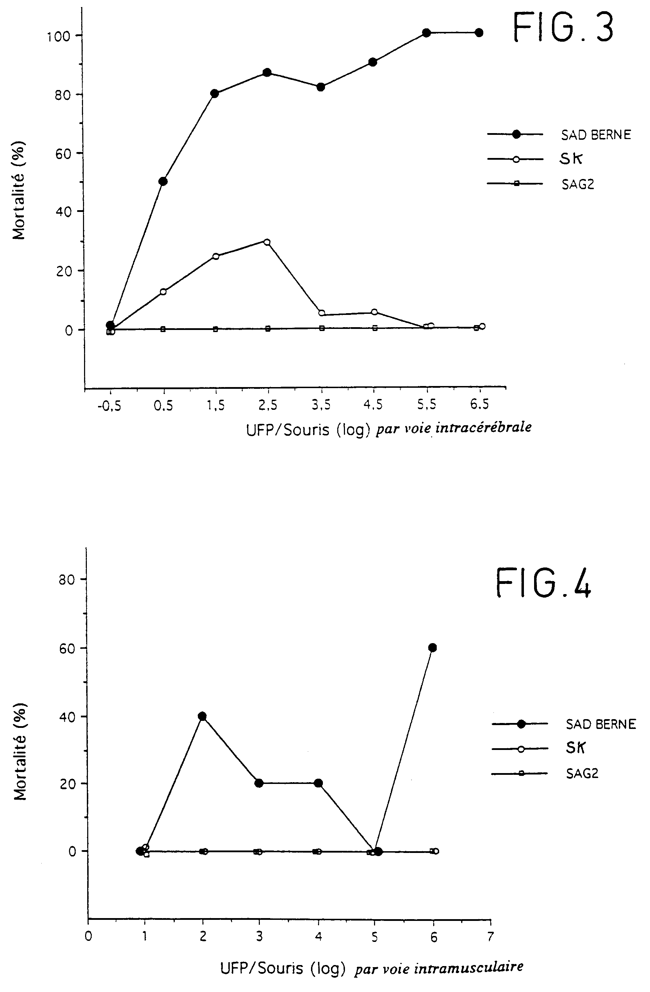

Les résultats sont reportés sur la figure 3 qui est un graphe donnant le % de mortalité (en ordonnées) en fonction de la quantité de mutant injectée par souris( en abscisses).

On constate que le mutant SAG2 ne provoque aucune mortalité alors que le mutant SK a un faible pouvoir pathogène résiduel.

### D- Pathogénie de SAG2 par voie intra-musculaire

On a répété le test ci-dessus mais par voie intra-musculaire. Les résultats obtenus sont reportés sur la figure 4 qui donne le % de mortalité (en ordonnées) en fonction de la dose administrée en UFP/souris (en abscisses).

On voit que SAG2 et le mutant SK ne provoquent aucune mortalité.

Les mutants selon l'invention peuvent être administrés à titre de vaccin vivant par tous les modes d'administration habituellement utilisés pour la vaccination et notamment par voie intra-musculaire ou orale. Les mutants sont avantageusement dilués dans un véhicule inerte pharmaceutiquement acceptable, tel que le sérum physiologique.

## Revendications

1. Vaccin antirabique avirulent, caractérisé en ce qu'il est constitué d'un mutant avirulent d'une souche SAD du virus rabique dont la glycoprotéine possède en position 333 un acide aminé naturel dont le codon diffère de ceux de l'arginine par au moins deux nucléotides.

2. Vaccin selon la revendication 1 caractérisé en ce que l'acide aminé en position 333 est l'acide glutamique.

3. Vaccin selon l'une des revendications 1 et 2, caractérisé en ce que le mutant est un mutant de la souche SAD Berne.

4. Vaccin selon l'une quelconque des revendications 1 à 3, caractérisé en ce que le mutant est le mutant déposé à la Collection Nationale de Cultures de Micro-organismes -INSTITUT PASTEUR (C.N.C.M) FRANCE le 9 juillet 1992 sous le N°I-1238.

5. Double mutant avirulent d'une souche SAD du virus rabique dont la glycoprotéine possède en position 333 un acide aminé naturel dont le codon diffère de ceux de l'arginine par deux nucléotides.

6. Double mutant avirulent selon la revendication 5, caractérisé en ce que l'acide aminé en position 333 est l'acide glutamique.

7. Double mutant avirulent selon l'une des revendications 5 ou 6, caractérisé en ce qu'il est un mutant de la souche SAD Berne.

8. Double mutant avirulent de la souche SAD Berne déposé à la Collection Nationale de Cultures de Micro-organismes (C.N.C.M) - INSTITUT PASTEUR - FRANCE le 9 juillet 1992 sous le N°I-1238.

9. Procédé pour l'obtention des doubles mutants selon l'une quelconque des revendications 5 à 8, caractérisé en ce qu'il consiste :
1/ à sélectionner à partir d'une souche SAD du virus rabique, les mutants qui ne sont pas neutralisés par un anticorps monoclonal neutralisant ladite souche SAD, mais ne neutralisant pas la souche TAG1 ;
2/ à isoler, par séquençage de la région 333 de la glycoprotéine des mutants sélectionnés à l'étape 1/, un mutant qui possède en 333 une lysine ;
3/ à préparer un anticorps monoclonal qui neutralise à la fois ladite souche SAD et le mutant obtenu à l'étape 2/, mais ne neutralise pas la souche TAG1 ;
4/ à procéder à une seconde sélection parmi les mutants obtenus à l'étape 1/ à l'aide de l'anticorps monoclonal préparé à l'étape 3/.

## Claims

1. An avirulent anti-rabies vaccine, characterized in that it consists of an avirvlent mutant of an SAD strain of the rabies virus, the glycoprotein of which possesses in position 333 a naturally occurring amino acid whose codon differs from those of arginine by at least two nucleotides.

2. A vaccine according to claim 1, characterized in that the amino acid in position 333 is glutamic acid.

3. A vaccine according to one of claims 1 and 2, characterized in that the mutant is a mutant of the SAD Berne strain.

4. A vaccine according to any one of claims 1 to 3, characterized in that the mutant is the mutant deposited in the Collection Nationale de Cultures de Micro-organismes - INSTITUT PASTEUR (C.N.C.M.) FRANCE on 9th July 1992 under no. I-1238.

5. A double avirulent mutant of an SAD strain of the rabies virus, the glycoprotein of which possesses in position 333 a naturally occurring amino acid whose codon differs from those of arginine by two nucleotides.

6. A double avirulent mutant according to claim 5, characterized in that the amino acid in position 333 is glutamic acid.

7. A double avirvlent mutant according to one of claims 5 or 6, characterized in that it is a mutant of the SAD Berne strain.

8. A double avirulent mutant of the SAD Berne strain, deposited in the Collection Nationale de Cultures de Micro-organismes (C.N.C.M.) - INSTITUT PASTEUR - FRANCE on 9th July 1992 under no. I-1238.

9. A method of obtaining the double mutants according to any one of claims 5 to 8, characterized in that it consists in:
1/ selecting, from an SAD strain of the rabies virus, those mutants which are not neutralized by a monoclonal antibody neutralizing said SAD strain but not neutralizing the TAG1 strain;
2/ isolating, by sequencing of the 333 region of the glycoprotein of the mutants selected in step 1/, a mutant which possesses a lysine in position 333;
3/preparing a monoclonal antibody which neutralizes both said SAD strain and the mutant obtained in step 2/, but does not neutralize the TAG1 strain; and
4/ effecting a second selection from the mutants obtained in step 1/ with the aid of the monoclonal antibody prepared in step 3/.

## Patentansprüche

1. Avirulenter Tollwut-Impfstoff, dadurch gekennzeichnet, daß er aus einer avirulenten Mutante eines Stammes SAD des Tollwutvirus besteht, deren Glykoprotein an Position 333 eine natürliche Aminosäure aufweist, deren Codon sich durch mindestens zwei Nucleotide von den Codons des Arginin unterscheidet.

2. Impfstoff nach Anspruch 1, dadurch gekennzeichnet, daß es sich bei der Aminosäure an Position 333 um Glutaminsäure handelt.

3. Impfstoff nach einem der Ansprüche 1 und 2, dadurch gekennzeichnet, daß die Mutante eine Mutante des Stammes SAD Berne ist.

4. Impfstoff nach einem der Ansprüche 1 bis 3, dadurch gekennzeichnet, daß es sich bei der Mutante um die bei der Nationalen Sammlung von Mikroorganismen-Kulturen (Collection Nationale de Cultures de Micro-organismes, C.N.C.M), INSTITUT PASTEUR - FRANKREICH am 9.Juli 1992 unter der Nr. I-1238 hinterlegte Mutante handelt.

5. Avirulente Doppelmutante eines Stammes SAD des Tollwutvirus, deren Glykoprotein an Position 333 eine natürliche Aminosäure aufweist, deren Codon sich durch zwei Nucleotide von den Codons des Arginin unterscheidet.

6. Avirulente Doppelmutante nach Anspruch 5, dadurch gekennzeichnet, daß es sich bei der Aminosäure an Position 333 um Glutaminsäure handelt.

7. Avirulente Doppelmutante nach einem der Ansprüche 5 oder 6, dadurch gekennzeichnet, daß sie eine Mutante des Stammes SAD Berne ist.

8. Avirulente Doppelmutante des Stammes SAD Berne, die bei der Nationalen Sammlung von Mikroorganismen-Kulturen (Collection Nationale de Cultures de Micro-organismes, C.N.C.M) - INSTITUT PASTEUR - FRANKREICH am 9.Juli 1992 unter der Nr. I-1238 hinterlegt wurde.

9. Verfahren zur Herstellung der Doppelmutanten nach einem der Ansprüche 5 bis 8, dadurch gekennzeichnet, daß es darin besteht:
1/ aus einem Stamm SAD des Tollwutvirus die Mutanten zu selektionieren, die nicht durch einen monoklonalen Antikörper neutralisiert werden, der diesen Stamm SAD, jedoch nicht den Stamm TAG1 neutralisiert;
2/ durch Sequenzierung des Bereichs 333 des Glykoproteins der in Schritt 1/ selektionierten Mutanten eine Mutante zu isolieren, die an Position 333 Lysin aufweist;
3/ einen monoklonalen Antikörper herzustellen, der zugleich den Stamm SAD und die in Schritt 2/ erhaltene Mutante, nicht jedoch den Stamm TAG1 neutralisiert;
4/ unter den in Schritt 1/ erhaltenen Mutanten mit dem in Schritt 3/ hergestellten monoklonalen Antikörper eine zweite Selektion vorzunehmen.
